# EUROPEAN PATENT SPECIFICATION

(11) **EP 3 312 305 B1**
(45) Date of publication and mention of the grant of the patent: **07.06.2023**
(21) Application number: 17197470.2
(22) Date of filing: 20.10.2017
(51) Int. Cl.: C25B 1/26, A61K 33/20, C02F 1/467

(54) **HYPOCHLOROUS ACID PRODUCTION**
HYPOCHLORSÄURE HERSTELLUNG
PRODUCTION D'ACIDE HYPOCHLOREUX

(30) Priority: 21.10.2016 ZA 201607271
(43) Date of publication of application: 25.04.2018
(73) Proprietor: Ultrapure HOCI (Pty) Ltd., 7140 Western Cape (ZA)
(72) Inventor: NWOZOR, Augustine Junior, 7140 Western Cape (ZA)
(74) Representative: Gill Jennings & Every LLP

(56) References cited:
- EP-A1- 2 826 888
- WO-A1-2016/160668
- WO-A2-2015/082937
- CN-A- 101 223 885
- CN-B- 102 217 649
- JP-A- 2007 301 541
- US-B2- 8 871 278
- FEREIDOUN FORGHANI ET AL: "Effect of water hardness on the production and microbicidal efficacy of slightly acidic electrolyzed water", FOOD MICROBIOLOGY., vol. 48, 24 December 2014 (2014-12-24), pages 28-34, XP055457237, GB ISSN: 0740-0020, DOI: 10.1016/j.fm.2014.11.020

## Description

### TECHNICAL FIELD

This invention relates to the stabilizing of hypochlorous acid solutions.

### BACKGROUND

The following discussion of the background art is intended to facilitate an understanding of the present invention only. The discussion is not an acknowledgement or admission that any of the material referred to is or was part of the common general knowledge as at the priority date of the application.

When an aqueous chlorine solution is used for the purpose of disinfection or as antimicrobial agent, it is not the chlorine *per se* but the free chlorine molecules ("FCMs") that dissociate from the chlorine ion that provide the disinfectant or antimicrobial function. There are three FCMs, viz. HOCI, chlorine gas and hypochlorite. Of these three FCM's, HOCI has the most pronounced effect in mitigating the spread of dangerous microorganisms. Also, whereas HOCI is tasteless and odourless and completely safe for human use, the other two FCMs (chlorine gas and hypochlorite) have a strong chlorine odour and pose a potential risk when brought into contact with human or animal skin, or by inhalation.

The prevalence of these three FCMs is generally governed by the pH (acidity level) of the solution in which they are present. An acidic environment (below pH 4.5) favours chlorine gas (Cl₂) to be present, whilst a relatively more alkaline solution (pH above 5.5) favours bleach hypochlorite (OCI⁻). "Pure" HOCI (i.e. a HOCI solution substantially free of contaminants other than water) is generally only feasible between pH 4.5 to 5.5. With prior methods of manufacturing HOCI of which the Applicant is aware, it is thus imperative that any solution that includes HOCI be maintained between pH 4.5 and 5.5, to ensure that HOCI does not form chlorine gas or hypochlorite. This is complicated when HOCI is to be included in creams, lotions, excipients, or solutions, which have a dilutory effect and which may affect the pH and HOCI concentration of the resultant mixture or solution leading to the inadvertent increase of undesirable bleach or hypochlorite molecules.

There are various methods of manufacturing solutions enriched for HOCI of which the Applicant is aware, but all suffer from the disadvantage of either being expensive, non-scalable, not resulting in HOCI substantially free of bleach contaminants, resulting in HOCI solutions with pH ranges making them unsuitable for use on human or animal subjects, or resulting in solutions being excessively dilute and hence unusable, typically having achievable concentrations below 80 mg/L (w/v). To date, the Applicant is not aware of a method or process that can provide a stable aqueous hypochlorous acid solution having hypochlorous acid present in a concentration of more than about 120 mg/L (w/v) without significant amounts of contaminating chlorine gas or hypochlorite ions.

WO 2016/160668 discloses hypochlorous acid compositions and their use for treating inflammatory or immunological conditions. Fereidoun Forghani et al., "Effect of water hardness on the production and microbicidal efficacy of slightly acidic electrolyzed water", Food Microbiology, vol. 48, 24 Dec. 2014 reports the effect of water hardness on slightly acidic electrolyzed water production, properties and microbicidal efficacy. JP 2007/301541 discloses a method and apparatus for generating slightly acidic electrolyzed water with a pH region where hypochlorous acid can be stably present, and EP 2,826,888 discloses a diaphragmless electrolyzer that produces subacid hypochlorous acid water by electrolyzing diluted hydrochloric acid. WO 2015/082937 discloses an ultrapure hypochlorous acid, whilst CN 101223885 discloses a micro-electrolysis disinfectant and preparation method thereof.The following invention seeks to propose possible solutions, at least in part, in amelioration of the above shortcomings in the art. US 8,871,278 discloses the stabilizing of hypochlorous acid solutions prepared by electrolysis of NaCl solution by dissolved inorganic carbon (DIC) added in the form of bicarbonate or carbonate of an alkali or alkaline earth metal.

### SUMMARY OF THE INVENTION

Disclosed herein is a solution of hypochlorous acid and water comprising:
hypochlorous acid; and
at least one of magnesium chloride and strontium chloride.

Typically, the solution would comprise one of magnesium chloride and strontium chloride.

The solution may have chlorine gas present in an amount of less than 30% (w/v).

The solution may have hypochlorite present in an amount less than 5% (w/v).

The solution may comprise a hypochlorous acid concentration of greater than 100 milligrams per litre (w/v).

The solution may have a pH in a range of from 3.0 to 5.8, more preferably from 4.0 to 5.8, most preferably from 4.5 to 5.5.

The solution may contain about 120 mg/L (w/v) or more, 200 mg/L or more, about 300 mg/L or more, about 400 mg/L or more, about 500 mg/L or more, about 600 mg/L or more, about 700 mg/L or more, about 800 mg/L or more, about 900 mg/L or more, or about 1000 mg/L or more hypochlorous acid.

Alternatively, or in addition, the solution may contain about 1000 mg/L or less, about 900 mg/L or less, about 800 mg/L or less, about 700 mg/L or less, about 600 mg/L or less, about 500 mg/L or less, about 400 mg/L or less, about 300 mg/L or less, or about 200 mg/L hypochlorous acid. Thus, the solution can contain hypochlorous acid in an amount bounded by any two of the aforementioned endpoints.

The solution may be an aqueous HOCI solution.

The solution may be substantially free of chlorine gas.

As such, the solution may have chlorine gas present in the solution in an amount of less than 300000 mg/L, preferably less than 200000 mg/L, preferably less than 100000 mg/L, preferably less than 50000 mg/L, preferably less than 20000 mg/L, preferably less than 10000 mg/L, preferably less than 5000 mg/L, more preferably less than 1000 mg/L, most preferably less than 100 mg/L, most preferably less than 10 mg/L. The chlorine gas is preferably measured as a percentage of the total free available chlorine (FAC). FAC is a combined form of hypochlorous acid, OCI- (hypochlorite anion) and Cl₂ (dissolved chlorine gas) in aqueous solution.

The solution may also be substantially free of bleach (hypochlorite).

As such, the solution preferably comprises less than 50000 mg/L, more preferably less than 20000 mg/L, more preferably less than 10000 mg/L, more preferably less than 5000 mg/L%, more preferably less than 100 mg/L, more preferably less than 10 mg/L bleach and/or hypochlorous acid salts, particularly as a percentage of the total FAC.

The solution may be a stable solution, providing stabilized hypochlorous acid. By "stable solution" or "stabilised hypochlorous acid" is meant a predominantly aqueous solution of HOCI in water wherein the HOCI does not readily convert into either greater than 30% chlorine gas or greater than 5% hypochlorite within 3 months of production.

The solution is preferably stable for at least 3 months, more preferably at least 6 months, more preferably at least 9 months, even more preferably at least 12 months, most preferably at least 24 months.

In one particular embodiment, disclosed herein is a stable aqueous solution of hypochlorous acid, the solution having less than 30% chlorine gas (w/v) and less than 5% hypochlorite ions, and wherein the solution comprises hypochlorous acid at a concentration of greater than 200 milligrams per litre.

The presence of magnesium chloride and strontium chloride may have resulted from the employment of magnesium carbonate and strontium carbonate, respectively, in producing the stable hypochlorous acid solution. Such production may have been in accordance with the method of the invention hereinafter described.

ACCORDING TO THE INVENTION THERE IS PROVIDED a method of producing a solution of hypochlorous acid and water ("the product solution") having a pH in a range of from 4.5 to 5.5 and a HOCI concentration of greater than 100 mg/L (w/v) and of 400 mg/L (w/v) or less, the method including subjecting a solution of hydrochloric acid and water ("the reaction solution") to electrolysis and thus obtaining HOCI in an electrolysis reaction; wherein magnesium carbonate (MgCO₃) or strontium carbonate (SrCO₃) is added to the product solution, the MgCO₃ or SrCO₃ being added to the product solution in a range of increments 60 g to 90 g of MgCO₃ or SrCO₃ per 100 L of product solution; and wherein the subjecting of the reaction solution to electrolysis is effected under a pressure greater than the hydrostatic pressure of the reaction solution, the pressure being maintained for the duration of the electrolysis reaction, wherein when MgCO₃ is used, the pressure is in a range of 5 kPa to 100 kPa; and when SrCO₃ is used, the pressure is in a range of 0.1 kPa to 2 kPa.

The product solution is a stable hypochlorous acid solution.

The reaction solution may comprise HCl in a concentration of from 2% to 12%, more preferably 5% to 10%, most preferably 6% to 9% v/v.

The reaction solution may have a pH in the region of 0.40 to 1.

The reaction solution may, in particular, comprise food-grade hydrochloric acid in reverse-osmosis (RO) water. The water may have a total dissolved solids (TDS) content of less than 5 mg/L.

The product solution may comprise magnesium chloride or strontium chloride in solution, depending on which of MgCO₃ and SrCO₃ was used.

If magnesium carbonate is used, the pressure may be in a range of 20 kPa to 40 kPa, most preferably 30 kPa. If strontium carbonate is used, the pressure may be in the range of 0.8 kPa to 1.2 kPa, most preferably 1.0 kPa.

The pressure would typically be exerted on the reaction solution. It must be appreciated that this does not mean that the pressure is not inherently also applied to the product solution, as the product solution is formed while subjecting the reaction solution to electrolysis.

The method of producing the stabilised hypochlorous acid solution of the invention therefore, essentially, proceeds according to the following equation which incorporates the electrolysis reaction: 2HCl + H₂O + electrical energy + pressure → HOCl + HCl + H₂ (g) (discounting here the involvement of MgCO₃ or SrCO₃).

The method may be performed in a pressurisable electrolysis chamber.

In one embodiment of the invention the method may be performed continuously, by continuously feeding, as in inflow to the electrolysis chamber, fresh reaction solution to the electrolysis chamber, subjecting the reaction solution to electrolysis in the electrolysis chamber, and continuously withdrawing, as an outflow from the electrolysis chamber, product solution from the electrolysis chamber. In such an embodiment, the pressure may be generated by throttling the outflow. Thus, the outflow may be restricted relative to the inflow.

Use of the pressurisable electrolysis chamber, or performance of the method generally, is of course not limited to continuous operation. The method may alternatively be operated in batch or in semi-batch (i.e. semi-continuous) fashion.

Through controlled pressure within the electrolysis chamber, most of the formed HCl is hydrolyzed into HOCI and H₂ (g). The Applicant has shown that it is possible to produce a stabilised HOCI solution having a concentration of greater than 200 mg/L using the method of the invention, up to as high as 400 mg/L, even as high as 1000 mg/L, with minimal contaminating bleach (hypochlorite) or chlorine gas molecules present in the final solution.

Advantageously, the Applicant has found that the addition of pressure to the electrolysis reaction results in HOCI being generated not only at the desired concentration (i.e. concentrations greater than 100 mg/L, even higher than 200, 400, or 1000 mg/L) but also at a more favourable pH level (i.e. down to pH 3.0), with minimal contaminants (e.g. disinfection by-products such as chlorate) present, as described hereinbefore.

The MgCO₃ or SrCO₃ is added in a range of 60 g to 90 g of MgCO₃ or SrCO3 per 100 L of product solution to be produced, for example 68 g of MgCO₃ or SrCO₃ per 100 L of product solution or 75 g of MgCO₃ or SrCO₃ per 100 L of product solution. In other words, the method may include adding from 60 g to 90 g of MgCO₃ or SrCO₃ to the reaction solution for every 100 L of product solution to be produced.

For example, at start-up addition of MgCO₃ or SrCO₃ to the reaction solution in the range of 60 g to 90 g may take place before subjecting the reaction solution to electrolysis, e.g. by adding MgCO₃ or SrCO₃ in the range of 60 g to 90 g to the electrolysis chamber and thereafter introducing the reaction solution into the electrolysis chamber. Thereafter, further MgCO₃ or SrCO₃ in the range of 60 g to 90 g may be added to the reaction solution after each 100 L volume of product solution produced. Of course, these amounts may be adjusted in accordance with the ratio provided, i.e. 60 g to 90 g per 100 L. For example, 30 g to 45 g per 50 L produced, etc.

Quantification of MgCO₃ or SrCO₃ may be empirically determined with reference to quantification of HOCI in the product solution. As indicated herein, 68 g of MgCO₃ or SrCO₃ has been found to be suitable for a concentration of 380 mg/L or up to 400 mg/L of HOCI, and 75 g for a concentration of 450 mg/L of HOCI.

While not wishing to be bound by theory, the Applicant understands that higher concentrations of HOCI has concomitant higher concentrations of HCl molecules, thus requiring higher amounts of MgCO₃ or SrCO₃ to achieve pH buffering of the product solution, as herein described.

The electrolytic cell may be a single electrolytic cell. This may be suitable for small volume production, while double, triple or greater numbers of electrolytic cells may be used for continuous production of larger volumes.

The electrolytic cell may be employed as a batch, continuous or semi-continuous electrolysis reactor.

The electrolytic cell may have included therein a cathode (negatively-charged) electrode or anode (positively-charged electrode).

Both the cathode and anode may be titanium-based.

The cathode may be a titanium-based cathode coated with porous layers of a metal oxide catalyst.

The metal oxide catalyst may be ruthenium-based, particularly ruthenium oxide (RuO₂).

Usefully, no separating membrane is required within the cell to separate the two electrodes. This is an additional feature of the invention, i.e. that the electrolytic cell may omit a separating membrane.

Subjecting the reaction solution to electrolysis may include exposing the reaction solution to an electrical current in a range of from 1 to 5 Ampere (A).

Subjecting the reaction solution to electrolysis may include exposing the reaction solution to a voltage in a range of from 12 volts (V) to 21 V, more preferably from 15 V and 19 V, most preferably 18 V.

The electrolytic cell may be included in the pressurisable electrolysis chamber.

Also disclosed herein, there is provided a use of magnesium carbonate or strontium carbonate in producing a solution of hypochlorous acid and water ("the product solution").

Also disclosed herein, there is provided an antimicrobial solution comprising, in an aqueous solution, hypochlorous acid and magnesium chloride or strontium chloride.

The aqueous HOCI solution comprised by the antimicrobial solution may be a stable hypochlorous acid solution as disclosed herein.

The aqueous HOCI solution comprised by the antimicrobial solution may be prepared using the method of the invention.

Alternatively and/or additionally, the aqueous HOCI solution comprised by the antimicrobial solution may be substantially free of chlorine ions, and/or measurably free of other chlorine chemical species and Disinfection By-Products (DBP)such as chlorate and trihalomethane.

As described herein, the ultrapure hypochlorous acid solution disclosed herein ("stable hypochlorous acid solution") and as produced using the method of the invention ("product solution") may be buffered to a higher pH using magnesium carbonate (MgCO₃) or strontium carbonate (SrCO₃).

Surprisingly, the Applicant has found that magnesium carbonate and strontium carbonate, respectively, also stabilises the ultrapure hypochlorous acid manufactured using the method of the invention.

Buffering and concomitant stabilisation is achieved by adding 60 g to 90 g of MgCO₃ or SrCO₃ per 100 L of electrolysed HOCI, preferably 68 g MgCO₃ or SrCO₃ per 100 L of electrolysed HOCI, typically for a concentration of 380 mg/L HOCI, or preferably 75 g of MgCO₃ or SrCO₃ per 100 L of HOCI, typically for a concentration of 450 mg/L HOCI.

After buffering of the mixture, it is important that the HOCI is present at the same concentration (mg/L) as prior to the buffering. The Applicant has found, surprisingly, that there is no perceived negative shift in HOCI concentration following buffering of the ultrapure hypochlorous acid solution disclosed herein with MgCO₃ or SrCO₃.

The method of the invention includes the step of incrementally adding the MgCO₃ or SrCO₃ to the reaction after formation of the electrolysed HOCl. As such, as has been stated, a desired amount of, say, 68 g of MgCO₃ may for example be added to an empty production vessel before manufacturing starts for a first batch of, say 100 L, and then 68 g of MgCO₃ or SrCO₃ may be added after every 100 L of production. It is therefore important to buffer/stabilise the solution ahead of the commencement of the production process, as Applicant has found that the stabilisation process is not as effective when the MgCO₃ or SrCO₃ is added once the hypochlorous acid solution has been finally produced.

The method of the invention may be performed in a single electrolysis chamber comprising an electrode housing, the electrode housing in turn having included therein electrodes in the form of electrolysis plates.

The housings may be dimensioned and spaced from one another such that they define a channel through which electrolyte may be forced under pressure, thereby minimising the amount of electrolyte not in contact with the electrolysis plates.

As such, the plates may each be substantially planar and parallel to one another inside the anode housing.

The electrolysis plates may be spaced between 1 mm and 30 mm from each other, preferably between 5 mm and 20 mm, most preferably between 8 mm and 10 mm.

The electrolysis plates may be of titanium (Ti) and at least one electrolysis plate may be covered with a porous metal catalyst.

In one embodiment, both electrolysis plates may be covered with a porous metal catalyst.

The porous metal catalyst may be ruthenium dioxide (RuOz).

As such, the disclosure extends to an electrolysis chamber for use in manufacturing hypochlorous acid, the electrolysis chamber comprising at least two electrolysis plates substantially parallel to one another and defining at least one channel between the electrolysis plates through which electrolyte to be electrolysed can be forced under pressure.

The electrolysis chamber may comprise a generally cylindrical electrode housing for housing the electrolysis plates.

The electrode housing may be circular cylindrical.

As such, the plates may each be substantially planar.

The plates may be substantially parallel to one another inside the anode housing.

The electrode housing may include two apertures at an operatively lower end thereof for receiving circuit wires of an electrical circuit, each wire being in contact with the anode or cathode, thereby to power the electrolysis reaction.

The electrode housing further may include a feed port through which electrolyte may be fed into the electrolysis chamber.

The disclosure further includes an enclosure which snugly fits over the electrode housing and which, when in place, significantly precludes electrolyte from contacting areas within the chamber which are outside the channel defined between the electrolysis plates.

The enclosure may be demountably attachable to the electrode housing such that it is possible to increase the pressure exerted on the electrolyte within the chamber to levels greater than the hydrostatic pressure of the electrolyte.

The enclosure may include a product port, through which the hypochlorous solution generated by the electrolysis reaction of the invention may be released from the electrolysis chamber.

In one embodiment, the feed port of the electrode housing and the product port of the enclosure are substantially in axial alignment.

Also disclosed herein is the use of the hypochlorous acid solution disclosed herein, variants thereof, or pharmaceutical compositions containing such hypochlorous acid solution disclosed herein, as well as to the hypochlorous acid solution disclosed herein, variants thereof, or pharmaceutical compositions containing such hypochlorous acid solution disclosed herein for use, in the treatment or prophylaxis of: an ocular condition, such as an ocular condition selected from an ocular injury, an ocular infection, a condition of increased pressure inside the eye, degenerative conditions of the retina and optic nerve, a cataract, and infection such as bacterial conjunctivitis, trachoma, viral conjunctivitis, fungal infection of the eye, infection of the Meibomian gland (Meibomianitis), allergic conjunctivitis, corneal ulcer, corneal chemical burn, superior limbic keratoconjunctivitis (episcleritis), corneal viral infection, ocular herpes viral infection, corneal scarring, scleral ptergium, blepheritis, scleral laceration, corneal angiogenesis, cataract reversal, neovascular macular degeneration, corneal acute viral endothelitis; a dental condition, such as a dental condition selected from periodontitis, loose teeth, bad breath, bleeding gums; a skin condition such as a skin condition selected from acne, rosacea, impetigo, cellulitis, fungal infections of the skin for example tinea pedis, pityriasis versicolor, tinea versicolor and candidiasis, viral infections of the skin for example herpes fever blisters and zoster, nappy rash, psoriasis, eczema and pigmentation disorders of the skin, including post-inflammatory hyper pigmentation (PIH), or to improve skin hydration; wounds, including skin and flesh wounds, such as burns, pressure sores, chronic wounds, diabetic ulcers, elective surgery wounds for example abdominal surgery wounds, thoracotomy wounds, gynaecological wounds, plastic surgery wounds, ear nose and throat surgery wounds, orthopaedic surgery wounds and stoma therapy wounds, wounds and infection of those wounds following a bite injury, especially from a dog or human; scars and scarring, including the treatment of scar tissue such as keloid scarring, or prevention or reduction of scar formation; or grey hair; or for use as a disinfecting agent prior to providing an intravitreal injection of a ocular therapeutic or by intracameral, intrascleral, intra-corneal, subconjunctival sub-tenon injection of the same, or treatment of snake venom in the eye as a result of spitting venom into the eye, a neutralising effect on biological warfare (anthrax and botulism), a germicidal effect on anthrax and botulism as well as their spores and a neutralising effect on their toxins.

Specifically, also disclosed herein is a composition comprising:
a cosmetically, dermatologically, pharmaceutically, or physiologically effective amount of the stabilised hypochlorous acid solution disclosed herein; and
a cosmetically, dermatologically, pharmaceutically, or physiologically acceptable vehicle, wherein said composition improves the condition and/or aesthetic appearance of a biological surface.

The improvement in aesthetic appearance, when using the composition or the hypochlorous acid solution disclosed herein, comprises: reducing dermatological signs of chronological aging, photo-aging, hormonal aging, and/or actinic aging; preventing and/or reducing the appearance of lines and/or wrinkles; reducing the noticeability of facial lines and wrinkles, facial wrinkles on the cheeks, forehead, perpendicular wrinkles between the eyes, horizontal wrinkles above the eyes, and around the mouth, marionette lines, and particularly deep wrinkles or creases; preventing, reducing, and/or diminishing the appearance and/or depth of lines and/or wrinkles; improving the appearance of suborbital lines and/or periorbital lines; reducing the appearance of crow's feet; rejuvenating and/or revitalizing skin, particularly aging skin; reducing skin fragility; preventing and/or reversing of loss of glycosaminoglycans and/or collagen; ameliorating the effects of estrogen imbalance; preventing skin atrophy; preventing, reducing, and/or treating hyperpigmentation; minimizing skin discoloration; improving skin tone, radiance, clarity and/or tautness; preventing, reducing, and/or ameliorating skin sagging; improving skin firmness, plumpness, suppleness and/or softness; improving procollagen and/or collagen production; improving skin texture and/or promoting texturisation; improving skin barrier repair and/or function; improving the appearance of skin contours; restoring skin luster and/or brightness; minimizing dermatological signs of fatigue and/or stress; resisting environmental stress; replenishing ingredients in the skin decreased by aging and/or menopause; improving communication among skin cells; increasing cell proliferation and/or multiplication; increasing skin cell metabolism decreased by aging and/or menopause; retarding cellular aging; improving skin moisturisation; enhancing skin thickness; increasing skin elasticity and/or resiliency; enhancing exfoliation; improving microcirculation; decreasing and/or preventing cellulite formation; or any combinations thereof.

Also disclosed herein is a composition, cream, excipient, salve, lotion, oil, exfoliant, or emollient containing the hypochlorous acid solution disclosed herein and to a method of making such a composition, cream, excipient, salve, lotion, oil, exfoliant, or emollient.

Also disclosed herein is the use of a stabilised hypochlorous acid solution disclosed herein in the manufacture of a pharmaceutical composition, or medicament, for the treatment of the conditions listed hereinbefore.

### BRIEF DESCRIPTION OF THE DRAWINGS

Further features of the present invention are more fully described in the following description of several non-limiting embodiments thereof. This description is included solely for the purposes of exemplifying the present invention as defined by the claims.

The description will be made with reference to the accompanying drawing in which:
Figure 1A is a three-dimensional representation of an electrode housing of an electrolysis chamber;
Figure 1B shows another aspect of a three-dimensional representation of an electrode housing of an electrolysis chamber;
Figure 2A is a side view of an electrode housing of an electrolysis chamber;
Figure 2B shows a top plan view of an electrode housing of an electrolysis chamber;
Figure 3A is three-dimensional representation of an enclosure for an electrolysis chamber;
Figure 3B shows another aspect of a three-dimensional representation of an enclosure for an electrolysis chamber;
Figure 4 is a cross-sectional side elevation of a complete electrolysis chamber;
Figure 5A is a three-dimensional view of a complete electrolysis chamber;
Figure 5B shows another aspect of a complete electrolysis chamber;
Figure 6A, 6B, and 6C show three-dimensional, top, and side views of a base plate spacer.
Figure 7 is a graph showing free chlorine production using three different electrodes;
Figure 8 is a graph showing the effect of different electrodes on the formation of Disinfection By-Products (DBP's); Figure 9 is a graph showing the relationship of free chlorine vs. pH of the HOCI solution disclosed herein;
Figures 10, 11 and 12 show results of wound care cases where HOCI (350 mg/L) was applied as shown;
Figure 13 shows results of herpes keratitis of the cornea (normal and magnified images), before and after treatment with HOCI (350 mg/L).
Figure 14A shows results of acute viral endothelialitis: before HOCI (350 mg/L) treatment - vision 20/65 and 20/200;
Figure 14B shows results of acute viral endothelialitis: after 6 weeks HOCI (350 mg/L) treatment - vision 20/25 and 20/25; and
Figure 15 shows results of acute viral conjunctivitis: after 6 days of treatment with HOCI (350 mg/L).

### DESCRIPTION OF EMBODIMENTS

The following modes, given by way of example only, are described in order to provide a more precise understanding of the subject matter of an embodiment or embodiments.

Hypochlorous acid (HOCI) has a multitude of uses and attributes, including: (i) a pronounced antiseptic/antimicrobial action against pathogens, including biofilm, bacteria, viruses, and fungi; (ii) A reduction in the inflammatory immune response (less redness, pain and swelling) and a more responsive healing reaction to dermal stressors; (iii) accelerated wound healing time; and (iv) skin rejuvenation due to the ameliorating effects of HOCI (on skin) of causative agents usually associated with environmental damage, which is the main cause of visible skin ageing.

Previous studies have shown that HOCI is 80x more powerful as a biocide or antimicrobial against *E*. *coli* than hypochlorite. HOCI has been thought of mostly as a transient by-product of ubiquitous chlorine disinfectants because of the difficulty associated with isolating and purifying HOCI. The antimicrobial mode of killing employed by HOCI is by causing respiratory loss in bacterial cell membranes due to an irreversible reaction with sulphur- and haeme-containing membrane enzymes and structural proteins leading to cell death and non-viability.

Recent advances in the production of cleaner (purer) HOCI solutions have shown its microbicidal effects against bacteria (including spores), parasites, viruses and fungi when compared to other disinfectants. To date, no build-up of resistance against its use has been demonstrated. Sang *et al.* describe the effect of 20, 40 and 80 ppm HOCI against a myriad of pathogenic bacteria and fungi, including but not limited to various *Mycobacterium* species, Methicillin resistant *Staphylococcus aureus* and biofilm-producing *Pseudomonas.* Based on this study it is recommended that HOCI be used in 80 ppm strength and applied for one minute to disinfect areas such as feedlots, slaughterhouses, hospitals, restaurants, schools, transportation huts and public restrooms.

The effects of HOCI on biofilm have also been studied extensively. Biofilm is a protective measure of certain microorganisms whereby a protective layer is created surrounding these organisms. It is a source of contamination inside water lines and anywhere where conditions for the continuous growth of bacteria are favourable (humid, warm and dark environments). In medicine it is a serious concern as it delays wound healing. In dental hygiene plaque is considered to be biofilm. HOCI penetrates biofilm and destroys it as well as killing the microorganisms that are responsible for its formation. HOCI at 3 ppm applied for two minutes is effective in destroying most biofilms. In the oral cavity it has been observed that a stronger reaction occurs against Gram-negative than Gram-positive bacteria. It has been found that HOCI was not toxic to oral mucosa and had similar antimicrobial efficacy to chlorhexidine, which is the most commonly used antimicrobial ingredient in mouth rinse products at present. The safety of chlorhexidine as a mouth rinse ingredient has been questioned as it kills oral bacteria indiscriminately. Some of the "good" bacteria are responsible for relaxing blood vessels and their loss could be indicated in a higher incidence of heart attacks and stroke in people that use chlorhexidine-containing mouth rinse products. In other studies, HOCI was also found to be highly effective against bacteria (including *Mycobacteria),* viruses and fungi, at concentration of less than 80 ppm.

Of particular interest is how effective HOCI is against viruses. For Adenovirus type 4, Bacteriophage, MS2, Hepatitis A & B (HAV), Herpes virus type 1, HIV-1, Human and Animal Influenza (including H5N1 Avian Influenza) virus, Human *Norovirus* (Norwalk), *Murine Norovirus, Orthopoxvirus, Poliovirus type 1 and 2, and Rhinovirus,* it was shown that only 0.02 ppm HOCI was needed to destroy these viruses.

Slightly acidic hypochlorous acid water (also referred to as Slightly Acidic Electrolyzed Water or SAEW) has been tested against bacteria, fungi, yeasts and viruses at 27 ppm for one minute resulting in 99.999% of microorganisms tested against being killed.

The efficacy of SAEW has also been tested and compared with acidic anolyte water. SAEW has a comparatively powerful bactericidal activity at the same available chlorine concentrations as low pH acidic electrolyzed water and is effective against *S*. *enteritidis* (100% inactivation @ 4 ppm after 2 min) and *S*. *enteritidis* on egg shells (100% inactivation at 15 ppm after 3 min). The presence of organic loading has been shown to significantly reduce the antimicrobial potential of free chlorine molecules. This is an important consideration in their application, since where a high organic load is likely, a higher-strength solution or continual delivery is required to maintain a high level of disinfection.

The reduction in the inflammatory response and its specific immune response against pathogenic Gram negative bacteria further support the beneficial effect of HOCI on the skin. HOCI modulates inflammation through its effect on nuclear factor ₖB and activator protein-1 of monocytes. After chlorination of taurine by HOCI, taurine chloramine is mostly anti-inflammatory. HOCI also neutralizes various pro-inflammatory cytokines and chemokine (IL-1^{beta,} IL-2, IL-6). The effect of this suppression of inflammation is to reduce pain, redness and swelling and to limit the potential risk of PIH and scarring in the healing skin.

The process of tissue and dermal healing is also accelerated when HOCI is applied to the skin. Healing is promoted by regulating cytokines and growth factors. After chlorination of taurine by HOCI, taurine chloramine enhances healing. Enhanced healing also happens due to neutralisation and inhibition of pro-inflammatory factors. The successful wound healing effect of HOCI has been reported on a series of chronic leg ulcers. Chronic non-healing leg ulcers showed a rapid improvement in their healing rate, with reduction in pain. HOCI is the key in promoting healing in variety of wound types. Other skin disinfectants e.g. diluted bleach (Dakin's solution), povidone iodine (Betadine), H₂O₂ and citric acid are cytotoxic and impede wound healing.

There are two main reasons for ageing of the skin: chronologic ageing is due to the natural process of ageing and is responsible for 20% of the appearance of ageing in the skin; environmental factors play a far bigger role in ageing and make up most of the remaining 80% as to why skin looks old. HOCI has the following skin rejuvenation effects:
- Reduction in epidermal thinning
- Slower epidermal cell turn-over rate
- Reduction in DNA-repair protein (raises with aging)
- Inflammation in the skin is reduced
- Reduction in environmental damage
- Beneficial effect on atopic dermatitis (eczema improves with the application of HOCI to the skin)

Multiple reports of the safety and non-toxic nature of HOCI have been published. These studies all have the same outcome: HOCI is non-toxic and safe for use, even in pregnant and lactating women, provided that HOCI is not accompanied by its bleach-containing or chlorine gas contaminants. The majority of existing methods known to the Applicant produce impure HOCI, irrespective of the concentration of the HOCI solution.

In the human body, HOCI is made in phagocytic white blood cells as integral part of immune response. An oxidative burst process forms peroxide (H₂O₂), which is then transformed by myeloperoxidase in the following reaction to form HOCl:

H₂O₂ + Cl⁻ + H⁺ → HOCl + H₂O

This process forms uncontaminated HOCI, with no (toxic) bleach-molecule elements present in the reaction. However, the concentration of HOCI formed in the body is very low.

Adding sodium hypochlorite (NaOCI) or calcium hypochlorite (Ca(OCl)₂) to water.

NaOCl + H₂O → HOCl + NaOH

Ca(OCl)₂ + 2 H₂O → 2 HOCl + Ca(OH)₂

Both these reactions form bleach molecules together with the HOCI.

Acidifying sodium hypochlorite:
With HCl:

   HCl + 2NaOCl → HOCl + Cl₂ + Cl₃⁻ + OCl⁻
With CO₂ (g):

   CO₂ + NaOCl + H2O → NaHCO3 + HOCl

Both these reactions form bleach molecules together with the HOCI

Hydrolysis of chlorine gas:

- Cl₂ + H₂O → HOCl + H⁺ + Cl⁻.

HOCI then rapidly decomposes:

2HOCl → 2HCl + O₂

This results in a highly unstable HOCI-mixture

Electrolysis of a brine solution. Electrolyzing salt in water gives the following reaction:
Anode:

   2Cl- (aq) → Cl₂ + 2e-, Cl₂ + 2H2O = 2HOCl + 2H+ + 2e- (oxidation) and Cl₂ + 2H+ + 2e- = 2HCl (reduction)
Final anode reaction:

   2Cl₂ + 2H₂0 = 2HOCl + 2HCl
Final cathode reaction:

   2Na+ + 2H₂O + 2e- → 2NaOH + H₂ (g)
Overall:

   4NaCl + 4H₂O = (2HOCl + 2HCl) + (2NaOH + H₂)

Summary of salt electrolysis:
i. Anolyte is very acidic (pH 2 - 2.2)
ii. Mostly due to HCl presence (HOCl:HCl = 1:1)
iii. Anolyte also contains Cl₂, due to the low pH of the solution:
iv. Anolyte also contains un-electrolyzed salt (NaCl)
v. Strong alkaline catholyte NaOH (pH 11 - 12)
vi. Proton/electron balance ensures that very acidic anolyte matches very alkaline catholyte. However, the potential application of anolyte is limited because of its lower pH values (≤2.7). At this low pH, dissolved Cl₂ gas can be rapidly lost due to volatilisation, decreasing the bactericidal activity of the solution with time and adversely affecting human health and environment. Moreover, the high acidity of anolyte may cause the corrosion of equipment and consequently limit its practical application.

A process of which the Applicant is aware that is used to buffer the acidic pH of the anolyte from pH 2.2 to pH 4.0 - 5.8 (the pH-range that favours HOCI), is to add NaOH to the anolyte. The reaction HOCI + NaOH = NaOCI + H₂O results in the formation of bleach in the mixture, which is a highly unfavourable by-product as its toxicity reduces its potential use in pharmaceutical applications and also lessens the mixture's disinfectant efficacy.

A method of producing HOCI as an aqueous solution thereof with a concentration of greater than 100 mg/L (w/v), typically greater than 125 mg/L (w/v), up to 1000 mg/L, is described herein. A method of stabilizing the HOCI is also described, which does not measurably reduce the bio-available HOCI in the resultant mixture.

The manufacturing process makes use of a single food-grade active ingredient (hydrochloric acid) and reverse osmosis (RO) water and delivers 500 - 800 ppm (mg/L) of pure HOCI, through an electrochemical process. Apart from HOCI and hydrogen gas (H₂) (which is gassed off from the mixture), no other contaminants such as bleach or chlorine gas are formed. The HOCI occurs in solution in an aqueous (i.e. water) environment. This makes the resultant HOCI solution or product suitable for application over a wide spectrum of applications, as the HOCI is strong enough to not only exert its effect on bacteria, viruses and fungi, but also on their spores. HOCI also has an anti-inflammatory effect and affects wounds positively as well as accelerating the healing process. This makes it suitable for use on humans and animals, even as an ocular application without side effects commonly associated with antimicrobials of which the Applicant is aware.

Throughout this specification, the % of chlorine gas in the solution is preferably the percentage of total chlorine present as chlorine gas. It may be measured with a chlorinometer, for example the HI 96771 Hanna Instruments UHR photo chlorinometer, or by any other standard method.

As a result, the Applicant believes it advantageous that the method of the invention allows safer, easier, more stable hypochlorous acid to be produced at higher concentrations than previously achieved by methods of which the Applicant is aware.

Compositions disclosed herein can be provided in any pharmaceutically, cosmetically and/or dermatologically suitable form, preferably as a lotion or cream, but also in an anhydrous or aqueous base, as well as in a sprayable liquid form. Other suitable cosmetic product forms for the compositions disclosed herein include but are not limited to, for example, an emulsion, a cream, a balm, a gloss, a lotion, a mask, a serum, a toner, an ointment, a mousse, a patch, pomade, a solution, a spray, a wax-based stick, or a towelette. In addition, the compositions contemplated herein can include one or more compatible cosmetically acceptable adjuvants commonly used and known by the skilled practitioner, such as colorants, fragrances, emollients, humectants, preservatives, vitamins, chelators. Due to its microbial effects on pathogenic microorganisms, biofilm, wound healing and immune modulation, it becomes evident that HOCI holds many promises as a treatment product. It is important to produce (and keep stable) a solution that contains as pure as possible HOCI without its parent (bleach) molecules, as the latter have toxic effects on the human body and on the environment.

The manufacturing of HOCI through an electrochemical process is well known and the production of anolyte through the electrolysis of a salt (NaCl) solution forms the mainstay of these processes. A two-chamber electrolytic cell separated by a semi-permeable membrane is used to produce both anolyte (highly acidic with pH lower than 2.7 and which contains remnants NaCl and other by-products), as well as alkaline catholyte.

The production of ultrapure HOCI may improve the bactericidal activity with maximizing the use of hypochlorous acid, reduce corrosion of surfaces, and minimize human health and safety issues from Cl₂ off-gassing. In the process in accordance with one aspect of the invention as described herein, "ultrapure" HOCI is generated using a single chamber electrolytic cell through which an electrolyte consisting of food-grade hydrochloric acid (HCl: CAS No. 7647-01-0 Sigma-Aldrich) in reverse-osmoses (RO) water with Total Dissolved Solids (TDS) of less than 5 mg/L is passed. The electrolytic cell contains Titanium (Ti) anodes and cathodes electrodes that are coated with Ruthenium Oxide (RuO₂) and no separating membrane exists in the chamber, separating these two electrodes. When a direct electrical current is applied between the positive electrodes (anodes) and the negative electrodes (cathodes), electrochemical processes at the material electrode surface transforms the electrolyte (HCl/RO water mixture) into an activated 'metastable' state, exhibiting elevated chemical reactivity and resulting in the modification of molecular ionic structures. Titanium (Ti) electrodes coated with porous layers of a metal oxide catalyst (RuO₂) ²⁹ are used due to improved characteristics of stability, electrochemical reactivity, corrosion resistance and lifespan of the electrodes.

This reaction whereby HOCI is formed is governed by the chemical reaction 2HCl + H₂O → HOCl + HCl + H₂ (g). As HCl is a much stronger acid than HOCI (HCl pKa = -7, HOCl pKa = 7.5), it stands to reason that the HCl in the mixture would have a far greater acidifying role than HOCI. This is the reason why, when manufacturing high concentrations of HOCI (125 to 1000 mg/L) that the pH of the mixture tends to be very low (*circa* pH 1.6 - 1.9). Using the method of single chamber electrolysis of HCl in water, it is possible up to a concentration of 100 mg/L HOCI to manage the pH lowering effect of HCl by reducing the flow of the electrolyte through the chamber (WO 2015/082937 A2), leading to secondary hydrolysis of the HCl, forming more HOCI. Lowering the flow rate then leads to an increase in HOCl:HCl ratio, which raises the pH of the mixture. In the above WO 2015/082937 patent application it is described how the concentration of HOCI can be raised to no more than 100 mg/L at pH 4.5.

The ratio of free chlorine molecules in a water solution is pH-dependent and (according to the Nernst equation) dictates which free form of chlorine is most prevalent within a generated solution: Cl₂(g), HOCI or ClO⁻. Between pH 4.5 and 5.5, the free chlorine molecules are mostly HOCI, with only traces of OCI- present. Below pH 4.5, the mixture starts forming chlorine gas (Cl_{2 (g)}) and above pH 5.5, the ratio of OCI- to HOCI slowly increases. It is for this reason that it is advantageous to have a solution containing HOCI at pH 4.5 to 5.5.

The method and process described herein for manufacturing highly concentrated (100 - 1000 mg/L) HOCI solutions with low levels of contaminating molecules, while arriving at a pH of 4.0 - 6.0, involves two steps: the process or method to manufacture or produce high concentrations of HOCI and buffering/stabilizing the HOCI.

Ultra-high concentrations of HOCI (200 - 1000 mg/L) hold many advantages over lower concentrations (below 100 mg/L HOCI). Many studies refer to the effectiveness of HOCI at low concentrations below 80 mg/L. It has been shown that, at a concentration of 125 mg/L, HOCI is effective against bacterial pathogens and at a concentration of 250 mg/L, *Aspergillus* is killed in two minutes.

It is not possible to produce hypochlorous acid of a physiologically acceptable pH at concentrations of 100 mg/L or higher using the prior art manufacturing methods known to the Applicant. For example, an electrolysis process that makes use of a brine solution, (like sodium chloride) to manufacture hypochlorous acid inevitably results in the manufacture of highly acidic (pH 2.0 - 3.0) hypochlorous acid that is too acidic for therapeutic use. At such a low pH, there is a significant (up to 30%) presence in the solution of chlorine gas, which can produce toxic side effects like lung, skin and eye irritation when in contact with human tissues. In order to achieve a more favourable pH where chlorine gas is not present in the solution, the solution needs to be buffered chemically. By buffering the highly acidic mixture of hypochlorous acid, the salts of hypochlorous acid, which form as a result of the buffering process, will be present in the mixture. The most common salts of hypochlorous acid, which results from the buffering of highly acidic hypochlorous acid, are sodium hypochlorite (liquid bleach) and calcium hypochlorite (solid bleach), both of which are undesirable in a topical treatment formulation for a product used to treat human skin and particularly human eyes. Their presence is associated with the development of side effects like eye or skin irritation, sensitivity, allergic reactions and lung irritation.

It should also be noted that the most commonly known method of electrolysis of water using sodium chloride produces not only a highly acidic mixture (anolyte) of hypochlorous acid, hydrochloric acid and chlorine gas but also one in which the concentration of the hypochlorous acid is usually high (140 - 2500 mg/Litre). Too high a concentration of hypochlorous acid can irritate human tissues. However, simply diluting the resulting mixture of hypochlorous acid and chlorine gas to reduce the concentration does not change the pH of the mixture. In fact, the dilution necessary to reduce the concentration of hypochlorous acid to avoid toxicity (this could be in the order of 1:500 dilution) is such that the concentration of hypochlorous acid in the diluted mixture will become too low and ineffective. Such small concentrations of hypochlorous acid solution have been found to be ineffective for the treatment of bacterial conjunctivitis. For example, if an anolyte mixture of 1000 mg/L of hypochlorous acid/Cl₂ -mixture is diluted by a factor of 1:500, the concentration of hypochlorous acid will only be 2 mg/L of hypochlorous acid/Cl₂ -mixture. The pH of the mixture is still very low, there will still be chlorine gas present in the mixture, which, if used, will cause tissue irritation

An effective electrolyser, also referred to an electrolysis chamber herein, requires consideration of the following variables, in which the Applicant has done much experimentation to arrive at the chamber, method, compositions of matter, and solution disclosed herein:

### 1.Electrical efficiency

This refers to the minimum voltage that is needed to apply across the anode/cathode without heat starts to develop in the electrolyte. Measuring the temperature in the electrolyte before it enters the electrolytic chamber and comparing the temperature of the electrolysed mixture, provides for the most efficient electrical tension (voltage V) to be applied to the chamber. As shown in Table 1, at a fixed electrical current (5 Amp) the Applicant found the following:

**Table 1. Voltage determination using magnesium carbonate - fixed Amperage (linear power source) - 5 A**

| Voltage (V) | Pre Electrolyzed Temperature | Post Electrolyzed |
|---|---|---|
| | (degrees C) | Temperature (degrees C) |
| 16 V | 14.8 | 15.2 |
| 17 V | 14.8 | 15.7 |
| 18V | 14.9 | 16.3 |
| 19 V | 14.9 | 16.9 |
| 20 V | 14.9 | 17.3 |
| 21 V | 14.9 | 17.5 |

**Table 2. Voltage determination using strontium carbonate - fixed Amperage (linear power source) - 5 A**

| Voltage (V) | Pre Electrolyzed Temperature | Post Electrolyzed |
|---|---|---|
| | (degrees C) | Temperature (degrees C) |
| 16 V | 14.8 | 14.9 |
| 17 V | 14.8 | 15.0 |
| 18 V | 14.9 | 15.3 |
| 19 V | 14.9 | 16.5 |
| 20 V | 14.9 | 17.3 |
| 21 V | 14.9 | 17.5 |

From the above, it was found that a fixed voltage of 18V would be most beneficial for electrolysis as it is the maximum voltage that can be applied without an exothermic reaction (heat developing reaction) that would lead to unneeded energy wastage.

### 2. Electrolysis Cell Design

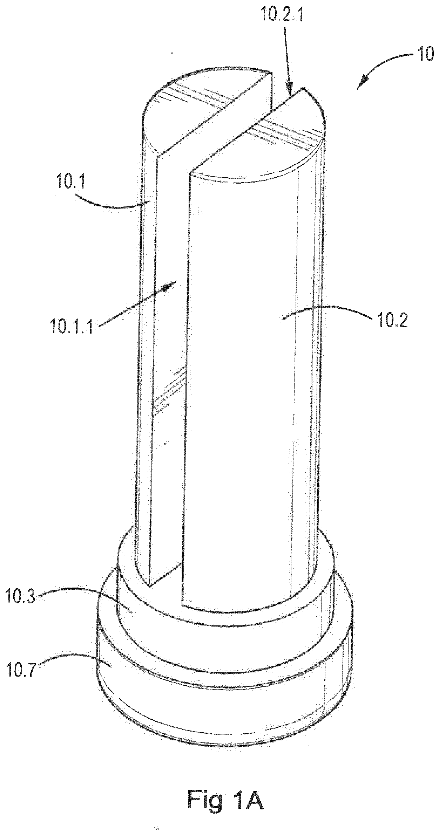

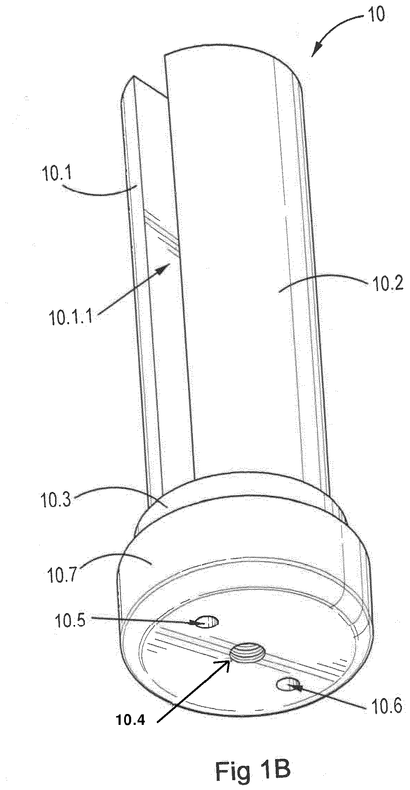

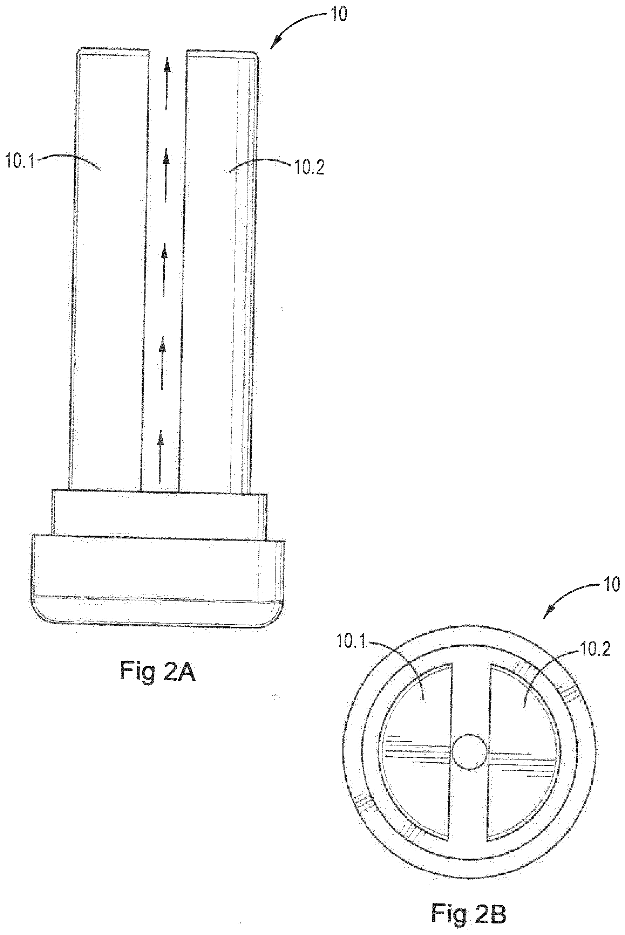

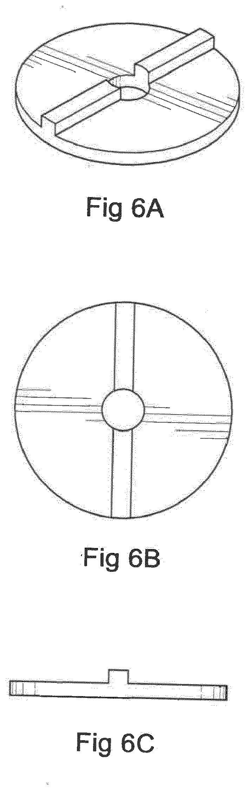

An electrolytic chamber's efficiency depends on the total surface area of the electrolytic (electrode) plates, viz. the electrical current-area that can affect the electrolyte. Applicant has found that it is important that no electrolyte be allowed to pass behind the electrodes where no electrical current exists. The invention extends to an electrolytic chamber, best shown in Figures 1 and 2. Figure 1A shows a circular cylindrical electrode housing indicated generally by reference numeral 10. The electrode housing 10 includes two electrodes in the form of electrolysis plates 10.1 and 10.2. The electrolysis plates rest on a spacer 10.3, best seen in Figures 6A, 6B, and 6C, which in turn rests on a base 10.7. The design of the electrode housing 10 allows for electrolyte (shown indicatively in Figure 2A using arrows) to only pass between facing surfaces 10.1.1 and 10.2.1 of the electrode plates 10.1 and 10.2, best shown generally in Figures 1A and 1B.

The internal design of the electrolytic chamber allows for the electrolysis plates 10.1 and 10.2 to be spaced closely to one other, substantially parallel, and allowing the electrolyte to only pass through the space provided. Very little electrolyte can pass between the outer periphery defined by the electrode plates 10.1, 10.2 and an inner surface of the closure 12. The facing surfaces 10.1.1 and 10.2.1 of the electrolysis plates 10.1, 10, 2 are, when using magnesium carbonate, at a spacing 8 mm to 10 mm from one another, with an acceptable range of 5 mm to 15 mm. When using strontium carbonate, the spacing is 0.8 mm to 1 mm, with an acceptable range of 0.5 mm to 1.5 mm

Apertures 10.5 and 10.6, defined on the bottom of the electrode housing, are for receiving positive and negative DC power cables for direct coupling to the anode and cathode, as appropriate.

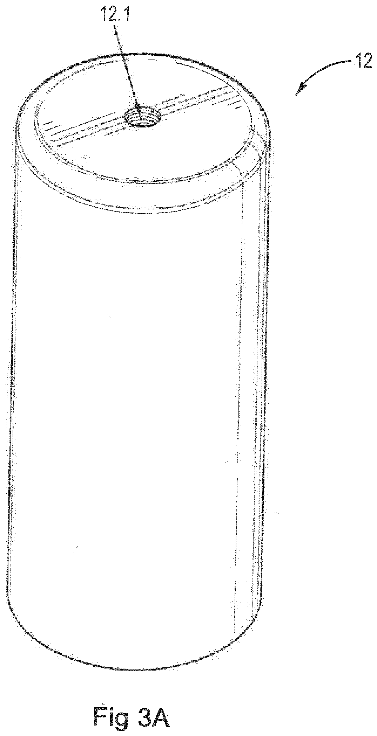

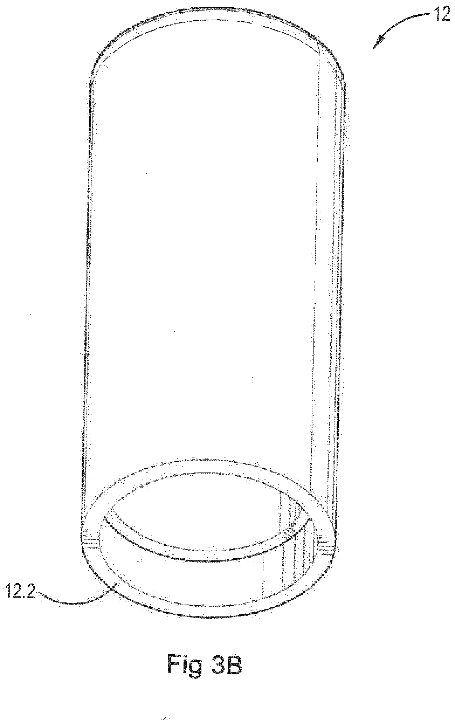

Figures 3A and 3B show an external electrolysis chamber closure 12. The closure 12 is circular cylindrical and is dimensioned to slide snugly over the internal electrode housing 10 and to abut the peripherally extending lower collar 10.7 of the anode housing 10 with a lower surface 12.2 thereof, when fully deployed over the electrode housing 10. The electrode housing 10 includes a centrally located annulus 10.4 for receiving electrolyte and in one embodiment the annulus 10.4 is threaded to allow for high pressure hoses (not shown) to be connected in fluid flow connection with an interior of the electrolysis housing 10. A corresponding annulus 12.1 is located on the external housing 12, longitudinally spaced from the annulus 10.4 contained in the anode housing 10 when the external housing 12 is placed over the electrode housing 10. The annulus (or port) 12.1 may be threaded to allow for high pressures hoses to be connected allowing for the exit of the hypochlorous acid solution disclosed herein. In this way, electrolyte can be forced from one end of the housing through annulus 10.4, under pressure, past the electrolysis plates 10.1 and 10.2 and out the corresponding, opposed annulus 12.1 contained in the external housing 12. Applicant has found that this leads to greatly increased concentrations of hypochlorous acid being created during the electrolytic process.

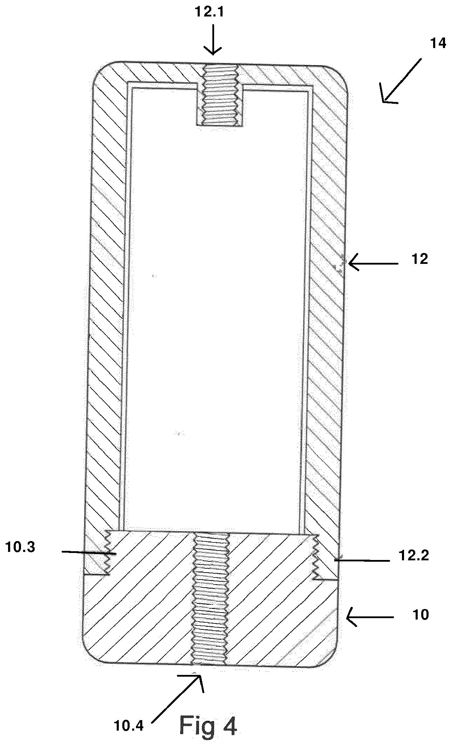

As shown in Figure 4, to contain and maintain the pressure within the assembled electrolytic chamber 14, the collar 10.3 is threaded, with complementary threads within an inner surface of the lower surface 12.2 of the closure 12.

The electrolytic plates are made of titanium (Ti) and covered with ruthenium dioxide RuO₂. The type of porous metal catalyst cover (RuO₂) is of utmost importance for the following reasons:

RuO₂/Ti produces a significantly higher concentration of free chlorine when compared to iridium dioxide (IrO₂/Ti) and platinum/Ti (Pt/Ti). RuO₂-coated electrodes are particularly attractive because of their remarkable electro catalytic properties, whereas IrO₂ are more stable and have a longer service life. RuO₂/Ti electrodes produced double the amount of free chlorine when compared to IrO₂/Ti in a study that compared different electrode materials when producing free chlorine.

Electrolysis is performed under pressure, usually 1.0kPA, to yield increased production of hypochlorous acid.

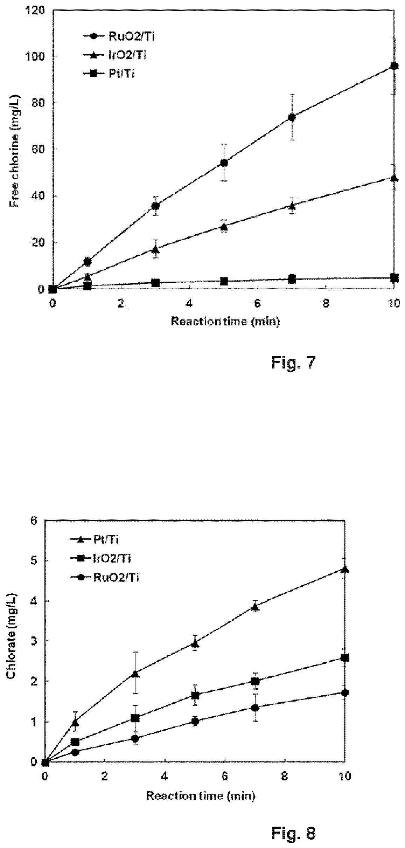

As shown in Figure 7, RuO₂ produces the fewest DBP's when compared to the above electrode materials. This study compared the levels of chlorate formation on the three tested electrodes. Chlorate is a neurotoxin and causes haemolytic anaemia by destroying red blood cells. Therefore, the World Health Organisation (WHO) has proposed a drinking water guideline concentration of 700 g/L.

From the graph in Figure 8 it can be seen that the use of RuO₂ covered electrodes produced the least DBP's when free chlorine is produced.

### 3. The electrolyte

### Electrolyte purity

The electrolyte should be prepared using RO or de-ionized water and food-grade hydrochloric acid (HCl: CAS No. 7647-01-0). It is important that the electrolyte is as clean as possible (free from contaminants other than the reactants water and HCl), as this prevents the formation of undesirable trihalomethane (THM) and haloacetic acids (HAA). The HCl should be free of contaminants like arsenic and other metals, as well as organic pollutants. Measurement of the total dissolved solids (TDS) in the prepared water should be performed and should be below 5 mg/L, but preferably below 2 mg/L. The type of electrode does not influence the formation of THM and HAA. It is therefore imperative that RO water and not tap water is used to produce ultrapure HOCI. In WO2015/082937A2 it is mentioned that pure HOCI can be manufactured using tap water, but in the Applicant's experience this is not the case. Impurities in the water (chlorine derivatives and organic compounds) lead to the formation of THM and HAA which, together with chlorates, give the preparation a distinct and undesirable chlorine smell. Using RO water, food grade HCl and RuO₂ electrodes, leaves an odourless pre-stabilised HOCI mixture.

### Automated pre-mixing

Pre-mixing RO water with food grade HCl is an automated process and is controlled by a pH probe inside the pre-mix tank that provides pH readings to a processor (Tekna PR40D Din Rail pH Controller, pH range 0 - 14) and an acid pump (Tekna AKS Solenoid metering pump).

### Current

Once the desired effective voltage was established (in this embodiment, 18 V), different strengths of HCl mixture were tested against fixed amperage of 2,5A until the most efficient HOCI production was performed, using electrolysis plates that were, in using magnesium carbonate, 10 mm apart and, in using strontium carbonate, 1mm apart. The results are shown in Tables 3 and 4, below.

**Table 3 - Using magnesium carbonate.**

| pH of water/HCl mixture | HOCl concentration mg/L |
|---|---|
| 1.0 | 185 |
| 0.90 | 210 |
| 0.80 | 235 |
| 0.70 | 325 |
| 0.60 | 315 |
| 0.50 | 290 |
| 0.40 | 295 |

**Table 4 - Using strontium cabonate.**

| pH of water/HCl mixture | HOCl concentration mg/L |
|---|---|
| 0.2 | 185 |
| 0.15 | 210 |
| 0.10 | 235 |
| 0.08 | 325 |
| 0.06 | 315 |
| 0.04 | 290 |
| 0.02 | 295 |

### 4. Pressure inside the electrolysis chamber

From the reaction: 2HCl + H₂O + electrical energy → HOCl + HCl + H₂ (g), it can be seen that hydrogen gas (H₂) is formed at the electrolysis rate that HOCI (and HCl) is formed. The gassing-off of H₂ forms vacuolisation at the plate-electrolyte interface, which would diminish the efficacy of the electrochemical reaction by means of which HOCI is produced. Applicant tested the efficacy of HOCI-production at various pressure settings inside the electrolysis chambers. Restricting the outflow of electrolysed solution and monitoring the pressure on an analogue pressure meter mounted on the electrode, allows one to find the most effective pressure for the electrolysis reaction. From the values in Tables 5 and 6 it can be seen that, in the case of using magnesium carbonate, a pressure increase up to 30 kPa and, in the case of using strontium carbonate, a pressure increase of up to 1.0 kPa provides for the most efficient electrolysis process to be completed.

**Table 5 - Using magnesium carbonate:**

| Pressure (kPa) | [HOCl] (mg/L) |
|---|---|
| 10 | 270 |
| 20 | 295 |
| 30 | 310 |
| 40 | 310 |
| 50 | 310 |
| 60 | 300 |
| 70 | 302 |
| 80 | 295 |
| 90 | 290 |
| 100 | 290 |

| | |
|---|---|
| Fixed electrical energy and flowrate | |

**Table 6 - Using strontium carbonate:**

| Pressure (kPa) | [HOCl] (mg/L) |
|---|---|
| 0.2 | 270 |
| 0.4 | 295 |
| 0.6 | 310 |
| 0.7 | 310 |
| 0.8 | 310 |
| 0.9 | 300 |
| 1.0 | 302 |
| 1.2 | 295 |
| 1.4 | 290 |
| 1.6 | 290 |

| | |
|---|---|
| Fixed electrical energy (18V) and flowrate (50 l/h (liters per hour) | |

### 5. Stabilisation of HOCl

As an electrolysed-produced molecule, HOCI is more stable than sodium hypochlorite (NaOCI), which has to be prepared for use (diluted from a concentrated solution) on a daily basis. HOCI on the other hand and prepared in our way is stable for up to two weeks in an open container and up to three months in a closed-glass container (ICH-conditions). Glass that is protected from UV-radiation generally offers the best environment for the preservation of the solution. This is due to the inert nature of glass. UV protection is needed as HOCI is rapidly degraded by UV-light.

Manufactured HOCI in water is an acidic mixture. Acid strength is expressed as a pKa-value. pKa = -log¹⁰ Ka. The lower the pKa value, the stronger the acid. The electrochemical process 2HCl + H₂O + electrical energy → HOCl + HCl + H₂ (g) shows that for every one HOCI molecule that is formed, one HCl molecule is also produced. HCl (pKa < 0) is a much stronger acid than HOCI (pKa 7.5) and this is one of the reasons why the product that is formed is acidic. The other reason for an acidic HOCI-containing mixture is that some of the HCl in the electrolyte is not hydrolysed (the efficiency of the hydrolysis process determines how much HCl in the electrolyte is hydrolysed). It is therefore necessary to treat the mixture for two reasons: The mixture must be buffered to a more favourable pH 4.0 - 6.0, and the HOCI must be stabilised.

### Buffering of the acidic HOCl mixture

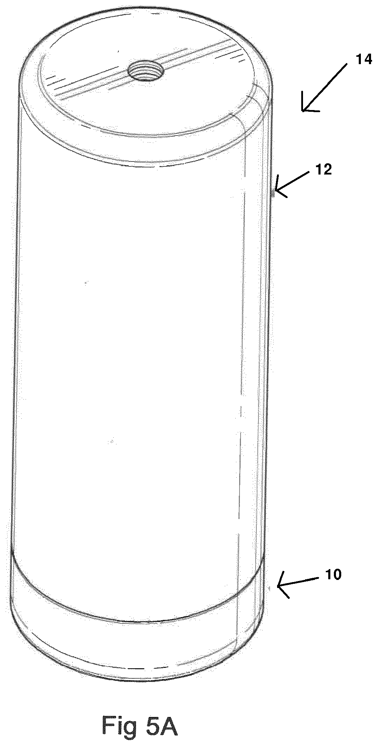

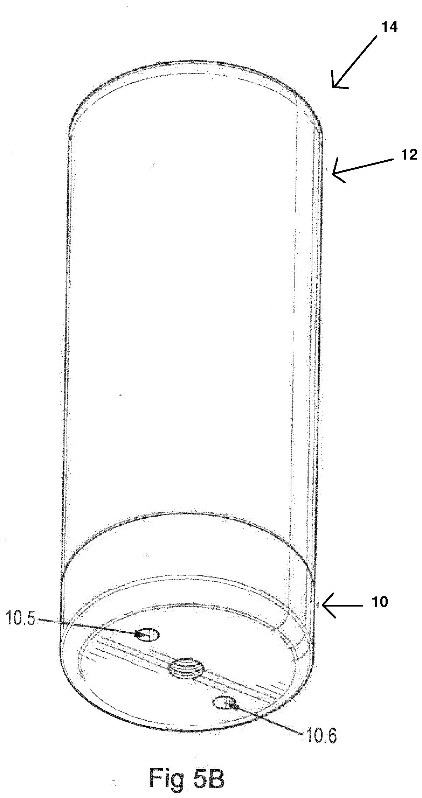

In any water solution where HOCl (and the other free chlorine molecules (Cl₂ and OCl⁻) are present, the prevalence of HOCI will be determined by the pH of the mixture. The graph in Figure 5 demonstrates the relationship between the free chlorine molecules in relationship with a changing pH.

It is therefore necessary to buffer an undesirably acidic mixture to a more favourable pH value (4.0 - 5.5), as a pH-value of below 4.0 will favour formation of Cl₂ gas and a pH-value of above 5.5 will favour the formation of OCl⁻ in the mixture.

### Buffering agent

Importantly, the buffering agent must avoid as far as possible the formation of HOCI salts (bleach), as the presence of bleach in the mixture will reduce the microbicidal effect of the mixture. In addition, bleach is toxic and will limit the potential use of the HOCI mixture in pharmaceutical applications. Furthermore, merely buffering the mixture to pH 4.0 - 5.5 will not increase the stability of the HOCI per se. It will merely ensure that the relationship of free chlorine molecules in the mixture will favour the prevalence of HOCI over Cl₂ and OCl⁻.

A buffering agent should also ideally act as a stabilizing agent. The buffering agent should also react with the HCl in the mixture, and not with the HOCI. Surprisingly, the Applicant has found that magnesium carbonate and strontium carbonate (MgCO₃ and SrCO₃) addresses these shortcomings.

MgCO₃ (CAS 7757-69-9) is not soluble in water but is soluble in HCl. MgCO₃ reacts with HCl in the following reaction: MgCO₃ + 2HCl → MgCl₂ + CO₂ + H₂O. In this reaction the HCl reacts with the CO₃²⁻ in the ratio 2 moles HCl: 1 mole CO₃²⁻. Applicant has found, surprisingly, that carbonate is an excellent buffering agent to remove excess HCl from the electrolysed HOCI product, thereby raising the pH to the desired level.

SrCO₃ (CAS 1633-05-02) is not soluble in water but is slightly soluble in HCl. SrCO₃ reacts with HCl in the following reaction: SrCO₃ + 2HCl → SrCl₂ + CO₂ + H₂O. In this reaction the HCl reacts with the CO₃²⁻ in the ratio 2 moles HCl: 1 mole CO₃²⁻. Applicant has found, surprisingly, that carbonate is an excellent buffering agent to remove excess HCl from the electrolysed HOCI product, thereby raising the pH to the desired level.

In one embodiment, the buffering reaction requires 68 g of MgCO₃ or SrCO₃ per 100 L of electrolysed HOCI (400 mg/L) and 75 g MgCO₃ or SrCO₃ per 100 L of HOCI 450 mg/L.

Following buffering of the mixture, it is important that the HOCI is present at the same concentration (mg/L) as before the addition of the buffering agent. Applicant has found that, advantageously, there is no negative shift in HOCI concentration when buffering HOCI-containing solutions with MgCO₃ or SrCO₃.

### 6. HOCl stabilisation

*Stabilisation through the electrochemical process.*

In an aqueous solution, hypochlorous acid partially dissociates into the anion *hypochlorite* ClO⁻ through the reaction 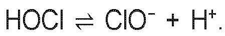 The reaction is unstable. Clustering of the water molecules, which in turn my lead to a more stable bond between the H₂O and HOCI, may result from the electrochemical production of HOCI.

The clusters of water molecules form an almost planar ring skeleton, made up of the O atoms and one H atom from each molecule, linked by hydrogen bonding to the next oxygen. The species with one water unit presents two almost equally stable conformers, *syn* and *anti.* Two stable structures have also been found for clusters with three and four molecules of water, one where the H⁺ atom of the HOCI molecule makes part of the ring, and another one in which the chlorine atom is in the ring. As the number of water-units increases, the corresponding clusters become more stable. The formation of hydrogen bonds and a partial proton transfer from HOCI to the water ring structure further strengthens the bond.

### Magnesium and strontium as stabilizing agents

HOCI therefore has the ability for H⁺ atom, as well as the Cl⁻molecules, to form covalent bonds. Without wishing to be bound be theory, Applicant postulates that the Mg²⁺ and Sr²⁺ molecules in the solution form covalent bonds with the Cl⁻ molecules of HOCI (with two molecules of HOCI bonding to one molecule of Mg²⁺ and Sr²⁺) and it is due to these bonds that the HOCI mixture becomes stable.

An observation that is worth mentioning is that the MgCO₃ or SrCO₃ must be added to the production process before formation of the electrolyzed HOCI in increments. Thus 68 g MgCO₃ or SrCO₃ was added to the empty production vessel before manufacturing starts and then add 68 g MgCO₃ or SrCO₃ after every 100 L of production. It is therefore important to buffer/stabilise the solution ahead of the production, as we have found that the stabilisation process is not as effective when the MgCO₃ or SrCO₃ is added once product has been formed. As an example, 680 g MgCO₃ or SrCO₃ added in this way produces 1000 litres of 400 mg/L HOCI at pH 5.7.

Stability has been tested under International Council for Harmonisation (ICH) conditions and found to be stable in dark glass containers for a period of up to 24 months. In addition, testing on plastic (PVC and LDPE) are in progress and shows stability up to and beyond 12 months.

An advantage of the current invention is the selective scrubbing of HCl in the manufactured product (HCl and HOCI are formed in a 1:1 ratio during manufacturing) with carbonate. This reduces the HCl component in the manufactured product, thereby raising the pH to an acceptable level for human and animal use. A further advantage is the stabilisation of the HOCI with Mg²⁺ or Sr²⁺.

Applicant has found that the stabilised hypochlorous acid solution disclosed herein finds use not only as a general antimicrobial solution, either by itself, when diluted, or when mixed with other compositions or excipients. In particular, the stabilised hypochlorous acid solution disclosed herein finds use in the treatment of: an ocular condition, such as an ocular condition selected from an ocular injury, an ocular infection, a condition of increased pressure inside the eye, degenerative conditions of the retina and optic nerve, a cataract, such as bacterial conjunctivitis, trachoma, viral conjunctivitis, fungal infection of the eye, meibomianitis, allergic conjunctivitis, corneal ulcer, corneal chemical burn, allergic conjunctivitis, superior limbic keratoconjunctivitis (episcleritis), corneal viral infection, ocular herpes viral infection, corneal scarring, scleral ptergium, blepheritis, scleral laceration, corneal angiogenesis, cateract reversal, neovascular macular degeneration, corneal acute viral endothelitis; a dental condition, such as a dental condition selected from periodontitis, loose teeth, bad breath, bleeding gums; a skin condition such as a skin condition selected from acne, rosacea, impetigo, cellulitis, fungal infections of the skin for example tinea pedis, pityriasis versicolor, tinea versicolor and candidiasis, viral infections of the skin for example herpes fever blisters and zoster, nappy rash, psoriasis, eczema and pigmentation disorders of the skin, including post inflammatory hyper pigmentation (PIH), or to improve skin hydration; wounds, including skin and flesh wounds, such as burns, pressure sores, chronic wounds, diabetic ulcers, elective surgery wounds for example abdominal surgery wounds, thoracotomy wounds, gynaecological wounds, plastic surgery wounds, ear nose and throat surgery wounds, orthopaedic surgery wounds and stoma therapy wounds, wounds and infection of those wounds following a bite injury, especially from a dog or human; scars and scarring, including the treatment of scar tissue such as keloid scarring, or prevention or reduction of scar formation; or grey hair; or for use as a disinfecting agent prior to providing an intravitreal injection of a ocular therapeutic or by intracameral, intrascleral, intra-corneal, subconjunctival sub-tenon injection of the same, the treatment of harvest sites for tissue transfer and tissue grafts (allografts and auto grafts, the neutralisation of toxins (staphylococcus, botulism, anthrax), as a disinfection agent against anthrax (*Bacillus anthracis), spores thereof,* and botulism-producing *Clostridium botulinum*

Also disclosed herein is not only the use of a hypochlorous acid solution disclosed herein in treating the following conditions, or in manufacturing a medicament for use in treating the following conditions, but also to a method of treating a condition selected from: an ocular condition, such as an ocular condition selected from an ocular injury, an ocular infection, a condition of increased pressure inside the eye, degenerative conditions of the retina and optic nerve, a cataract, such as bacterial conjunctivitis, trachoma, viral conjunctivitis, viral keratitis, fungal infection of the eye, meibomianitis, allergic conjunctivitis, corneal ulcer, corneal chemical burn, allergic conjunctivitis, superior limbic keratoconjunctivitis (episcleritis), corneal viral infection, ocular herpes viral infection, corneal scarring, scleral ptergium, blepheritis, scleral laceration, corneal angiogenesis, cateract reversal, neovascular macular degeneration; a dental condition, such as a dental condition selected from periodontitis, loose teeth, bad breath, bleeding gums; a skin condition such as a skin condition selected from acne, rosacea, impetigo, cellulitis, fungal infections of the skin for example tinea pedis, pityriasis versicolor, tinea versicolor and candidiasis, viral infections of the skin for example herpes fever blisters and zoster, nappy rash, psoriasis, eczema and pigmentation disorders of the skin, including post inflammatory hyper pigmentation (PIH), or to improve skin hydration; wounds, including skin and flesh wounds, such as burns, pressure sores, chronic wounds, diabetic ulcers, elective surgery wounds for example abdominal surgery wounds, thoracotomy wounds, gynaecological wounds, plastic surgery wounds, ear nose and throat surgery wounds, orthopaedic surgery wounds and stoma therapy wounds, wounds and infection of those wounds following a bite injury, especially from a dog or human; scars and scarring, including the treatment of scar tissue such as keloid scarring, or prevention or reduction of scar formation; or grey hair; or of disinfecting an area prior to providing an intravitreal injection of a ocular therapeutic or by intracameral, intrascleral, intra-corneal, subconjunctival sub-tenon injection of the same, the treatment of harvest sites for tissue transfer and tissue grafts (allografts and auto grafts, the neutralisation of toxins (staphylococcus, botulism, anthrax), as a disinfection agent against anthrax (*Bacillus anthracis), spores thereof* and botulism-producing *Clostridium botulinum,* the method comprising administering an effective amount of a solution disclosed herein or a disclosed herein to a subject in need thereof. Further uses are shown in Table 4, below.

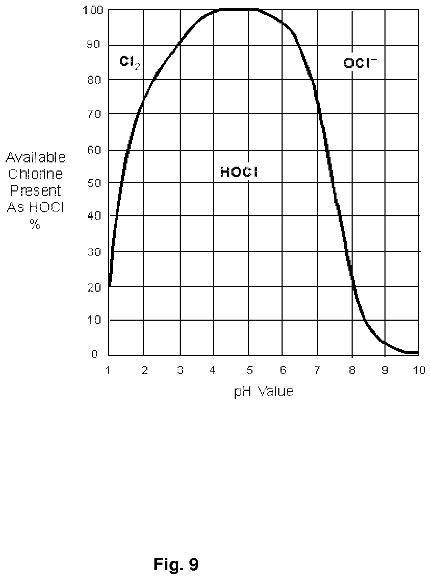

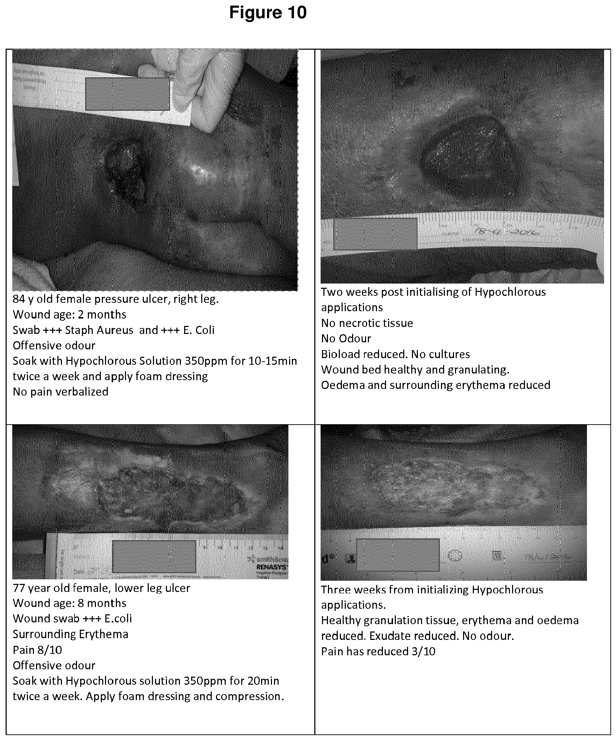

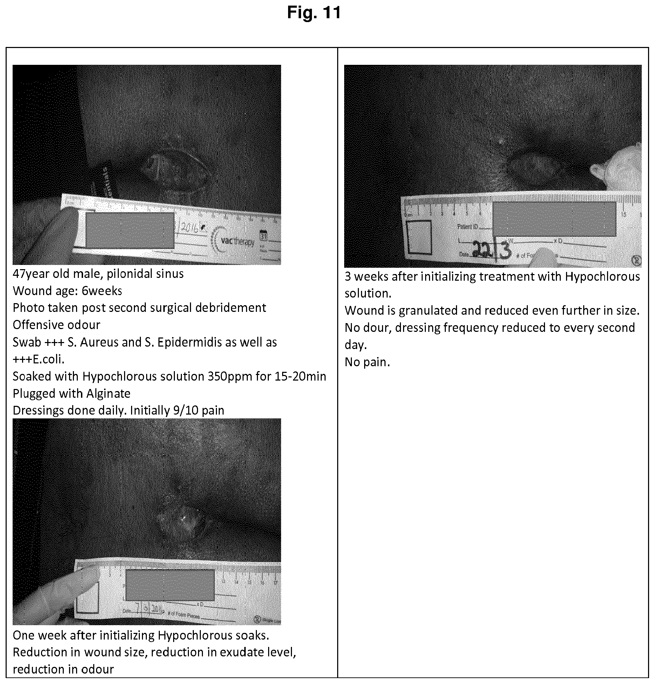

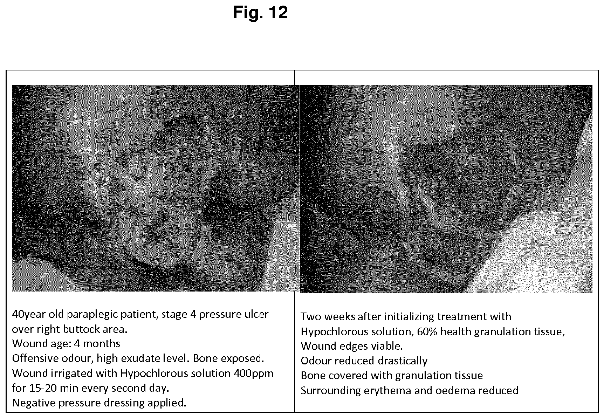

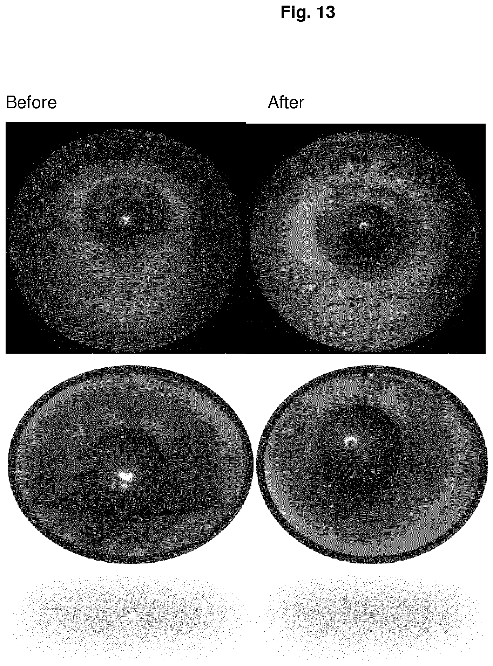

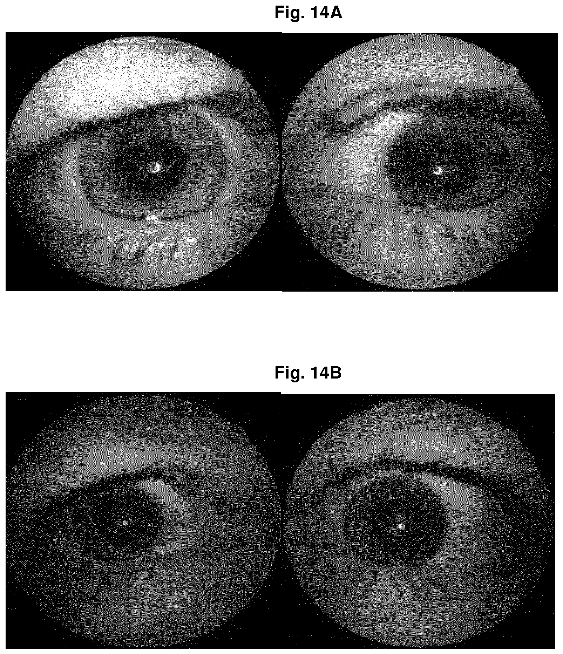

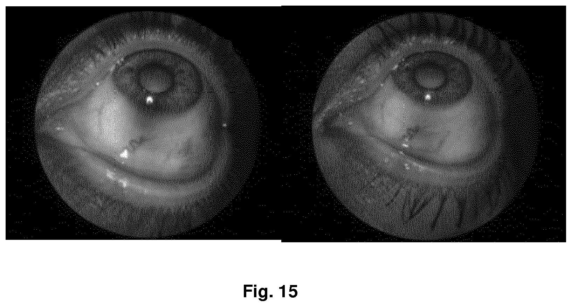

Results obtained with the hypochlorous acid solution disclosed herein, and/or compositions including the hypochlorous acid solution disclosed herein, are shown in Figures 9 to 15.

**TABLE 4: Applications of HOCI produced in accordance with one aspect of the invention**

| **PRODUCT** | **SECTOR** |
|---|---|
| Skincare, including cosmetic improvement of the skin, the treatment of acne, rosacea, eczema and furuncle (staphylococcal infection) | Beauty/Aftercare |
| | 200 ppm |
| Skincare: an aftercare for skin manipulation, e.g. after laser, dermabrasion and skin peeling procedures. Accelerates healing, reduces pain and prevents infection and post inflammation pigmentation disorders. | Professional Beauty Environment |
| | 250 ppm |
| Bacterial and fungal skin infections e.g. nappy rash, athlete's foot, groin infections and herpes skin infections. Other viral infections e.g. skin warts, papilloma virus and plantar warts. | Medical 250 - 400 ppm |
| Scalp Spray | Personal Care |
| | 250 ppm |
| Eye Drop | Health/Eye Care, OTC |
| | 200 ppm |
| Surgical Wound Wash | Health Care |
| | 400 ppm |
| Burn Wound Care | Health Care |
| | 400 ppm |
| Lower Leg Ulcers | Health Care, OTC |
| | 400 ppm |
| Mouth Wash | Health Care, OTC |
| | 200 ppm |
| Fruit and Vegetable spray | Food Industry/Agricultural |
| | 200 ppm |
| Neutralizing ammonia in chicken manure to make it more palatable when used in animal feed | Animal food industry |
| | 8 - 200 ppm |
| Red meat, fish and chicken meat spray | Food Industry/agricultural |
| | 200 ppm |
| Crop Spraying against Bacterial, Viral, Fungal disease | Agriculture |
| | 200 - 400 ppm |
| Facility reticulation water treatment for hospitals | Water supply Industry |
| | 2 ppm |
| Air conditioning tunnel misting | Mining Industry |
| | 400 ppm |
| Animal husbandry | Food production/agriculture |
| | 2 ppm |
| General Disinfectant | Restaurants, hospitals, paper producing industry, wine producing industry, food packaging, vegetable fogging in supermarkets |
| | 250 ppm |
| High Level Disinfectant | Hospitals, Sterilisation of instruments, fiber-optic scopes |
| Disinfection of milk parlors | Agriculture. |
| | 250 ppm |
| High Level Disinfectant | Hospitals |
| | 500 ppm |
| Various Veterinary Products | Veterinary Medicine, OTC |
| Food Production | Kitchens, restaurants, supermarkets |
| Fresh Fruit and Vegetables | Retail outlets |
| Meat Production | Prevention of food poisoning |
| Mine air conditioning | Treatment of mold infestation in cooling towers |
| Sick Building Syndrome Treatment | Building Industry - treatment of bacterial, fungal, and viral pathogens in HVAC systems |

It is to be appreciated that reference to "one example" or "an example" of the invention is not made in an exclusive sense. Accordingly, one example may exemplify certain aspects of the invention, whilst other aspects are exemplified in a different example. These examples are intended to assist the skilled person in performing the invention and are not intended to limit the overall scope of the invention in any way unless the context clearly indicates otherwise.

The use of words that indicate orientation or direction of travel is not to be considered limiting. Thus, words such as "front", "back", "rear", "side", "up", down", "upper", "lower", "top", "bottom", "forwards", "backwards", "towards", "distal", "proximal", "in", "out" and synonyms, antonyms and derivatives thereof have been selected for convenience only, unless the context indicates otherwise. The inventor(s) envisage that various exemplary embodiments of the subject matter can be supplied in any particular orientation and the subject matter is intended to include such orientations.

The use of the terms "a", "an", "said", "the", and/or similar referents in the context of describing various embodiments are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted.

Moreover, when any number or range is described herein, unless clearly stated otherwise, that number or range is approximate. Recitation of ranges of values herein are merely intended to serve as a shorthand method of referring individually to each separate value falling within the range, unless otherwise indicated herein, and each separate value and each separate sub-range defined by such separate values is incorporated into the specification as if it were individually recited herein. For example, if a range of 1 to 10 is described, that range includes all values there between, such as for example, 1.1, 2.5, 3.335, 5, 6.179, 8.9999, etc., and includes all sub-ranges there between, such as for example, 1 to 3.65, 2.8 to 8.14, 1.93 to 9, etc.

## Claims

1. A method of producing a solution of hypochlorous acid and water ("the product solution") having a pH in a range of from 4.5 to 5.5 and a HOCI concentration of greater than 100 mg/L (w/v) and of 400 mg/L (w/v) or less, the method including subjecting a solution of hydrochloric acid and water ("the reaction solution") to electrolysis and thus obtaining HOCI in an electrolysis reaction;
wherein magnesium carbonate (MgCO₃) or strontium carbonate (SrCO₃) is added to the product solution, the MgCO₃ or SrCO₃ being added to the product solution in a range of increments 60 g to 90 g of MgCO₃ or SrCO₃ per 100 L of product solution; and
wherein the subjecting of the reaction solution to electrolysis is effected under a pressure greater than the hydrostatic pressure of the reaction solution, the pressure being maintained for the duration of the electrolysis reaction, wherein when MgCO₃ is used, the pressure is in a range of 5 kPa to 100 kPa; and when SrCO₃ is used, the pressure is in a range of 0.1 kPa to 2 kPa.

2. The method according claim 1, wherein MgCO₃ is used, and the pressure is in a range of 20 kPa to 40 kPa, more preferably 30 kPa; or SrCO₃ is used, and the pressure is in a range of 0.8 kPa to 1.2 kPa, more preferably 1.0 kPa.

3. The method according to claim 2, which is performed continuously in a pressurisable electrolysis chamber, by continuously feeding, as in inflow, fresh reaction solution to the electrolysis chamber, continuously subjecting the reaction solution to electrolysis in the electrolysis chamber, and continuously withdrawing, as an outflow, product solution from the electrolysis chamber.

4. The method according to claim 3, wherein the pressure is generated by throttling the outflow.

5. The method according to any of claims 1 to 4, wherein subjecting the reaction solution to electrolysis includes exposing the reaction solution to an electrical current in a range of from 1 to 5 A and to a voltage in a range of from 12 V to 21 V, more preferably from 15 V to 19 V, most preferably 18 V.

6. The method according to any of claims 1 to 5, wherein the reaction solution comprises HCl in a concentration of from 5% to 10%, more preferably 6% to 9% v/v.

## Patentansprüche

1. Verfahren zur Herstellung einer Lösung aus hypochloriger Säure und Wasser ("die Produktlösung") mit einem pH-Wert im Bereich von 4,5 bis 5,5 und einer HOCI-Konzentration von mehr als 100 mg/L (w/v) und von 400 mg/L (w/v) oder weniger, wobei das Verfahren enthält eine Lösung von Salzsäure und Wasser ("die Reaktionslösung") einer Elektrolyse zu unterziehen und somit HOCI in einer Elektrolysereaktion zu erhalten;
wobei der Produktlösung Magnesiumcarbonat (MgCO₃) oder Strontiumcarbonat (SrCO₃) zugeführt wird, wobei das MgCO₃ oder SrCO₃ der Produktlösung in einem Bereich von Inkrementen von 60 g bis 90 g MgCO₃ oder SrCO₃ pro 100 L Produktlösung zugeführt wird; und
wobei das die Reaktionslösung einer Elektrolyse Unterziehen unter einem Druck erfolgt, der größer ist als der hydrostatische Druck der Reaktionslösung, wobei der Druck für die Dauer der Elektrolysereaktion aufrechterhalten wird, wobei, wenn MgCO₃ verwendet wird, der Druck in einem Bereich von 5 kPa bis 100 kPa liegt, und wenn SrCO₃ verwendet wird, der Druck in einem Bereich von 0,1 kPa bis 2 kPa liegt.

2. Verfahren nach Anspruch 1, wobei MgCO₃ verwendet wird und der Druck in einem Bereich von 20 kPa bis 40 kPa, vorzugsweise 30 kPa, liegt, oder SrCO3 verwendet wird und der Druck in einem Bereich von 0,8 kPa bis 1,2 kPa, vorzugsweise 1,0 kPa, liegt.

3. Verfahren nach Anspruch 2, das kontinuierlich in einer druckbeaufschlagbaren Elektrolysekammer durchgeführt wird, indem der Elektrolysekammer kontinuierlich frische Reaktionslösung als Zufluss zugeführt wird, die Reaktionslösung in der Elektrolysekammer kontinuierlich einer Elektrolyse unterzogen wird und Produktlösung als Ausfluss aus der Elektrolysekammer kontinuierlich abgezogen wird.

4. Verfahren nach Anspruch 3, wobei der Druck durch Drosselung des Ausflusses erzeugt wird.

5. Verfahren nach einem der Ansprüche 1 bis 4, wobei das die Reaktionslösung einer Elektrolyse Unterziehen enthält, die Reaktionslösung einem elektrischen Strom im Bereich von 1 bis 5 A und einer Spannung im Bereich von 12 V bis 21 V, vorzugsweise von 15 V bis 19 V, besonders bevorzugt 18 V, auszusetzten.

6. Verfahren nach einem der Ansprüche 1 bis 5, wobei die Reaktionslösung HCl in einer Konzentration von 5 % bis 10 %, vorzugsweise 6 % bis 9 %, v/v enthält.

## Revendications

1. Méthode de production d'une solution d'acide hypochloreux et d'eau (« la solution produite ») ayant un pH situé dans la plage allant de 4,5 à 5,5 et une concentration de HOCl supérieure à 100 mg/l (p/v) et de 400 mg/l (p/v) ou moins, la méthode comprenant la soumission d'une solution d'acide chlorhydrique et d'eau (« la solution réactionnelle ») à une électrolyse et ainsi l'obtention de HOCl dans une réaction d'électrolyse ;
dans laquelle du carbonate de magnésium (MgCO₃) ou du carbonate de strontium (SrCO₃) est ajouté à la solution produite, le MgCO₃ ou le SrCO₃ étant ajouté à la solution produite dans une plage d'incréments allant de 60 g à 90 g de MgCO₃ ou de SrCO₃ pour 100 litres de solution produite ; et
dans laquelle la soumission de la solution réactionnelle à une électrolyse est effectuée sous une pression supérieure à la pression hydrostatique de la solution réactionnelle, la pression étant maintenue pendant toute la durée de la réaction d'électrolyse, dans laquelle, quand du MgCO₃ est utilisé, la pression est située dans la plage allant de 5 kPa à 100 kPa ; et quand du SrCO₃ est utilisé, la pression est située dans la plage allant de 0,1 kPa à 2 kPa.

2. Méthode selon la revendication 1, dans laquelle du MgCO₃ est utilisé, et la pression est située dans la plage allant de 20 kPa à 40 kPa, plus préférentiellement de 30 kPa ; ou du SrCO₃ est utilisé, et la pression est située dans la plage allant de 0,8 kPa à 1,2 kPa, plus préférentiellement de 1,0 kPa.

3. Méthode selon la revendication 2, qui est effectuée en continu dans une chambre d'électrolyse pressurisable, par introduction en continu, sous la forme d'un courant d'entrée, de solution réactionnelle fraîche dans la chambre d'électrolyse, soumission en continu de la solution réactionnelle à une électrolyse dans la chambre d'électrolyse, et retrait en continu, sous la forme d'un courant de sortie, de solution produite à partir de la chambre d'électrolyse.

4. Méthode selon la revendication 3, dans laquelle la pression est générée par étranglement du courant de sortie.

5. Méthode selon l'une quelconque des revendications 1 à 4, dans laquelle la soumission de la solution réactionnelle à une électrolyse comprend l'exposition de la solution réactionnelle à un courant électrique situé dans la plage allant de 1 à 5 A et à une tension située dans la plage allant de 12 V à 21 V, mieux encore de 15 V à 19 V, tout spécialement de 18 V.

6. Méthode selon l'une quelconque des revendications 1 à 5, dans laquelle la solution réactionnelle comprend du HCl à une concentration de 5 % à 10 %, mieux encore de 6 % à 9 % v/v.
